# EUROPEAN PATENT APPLICATION

(11) **EP 4 289 807 A1**
(43) Date of publication of application: **13.12.2023**
(21) Application number: 22178515.7
(22) Date of filing: 10.06.2022
(51) Int. Cl.: C07C 1/24, C07C 7/144, C07C 11/04

(54) **ETHANOL BASED INTENSIFIED ETHYLENE PRODUCTION**

(71) Applicant: Nederlandse Organisatie voor toegepast- natuurwetenschappelijk onderzoek TNO, 2595 DA 's-Gravenhage (NL)
(72) Inventor: The designation of the inventor has not yet been filed
(74) Representative: Nederlandsch Octrooibureau

(57) **Abstract**

The invention concerns a process for converting ethanol to ethylene, comprising a catalytic dehydration step wherein ethanol is dehydrated to form ethylene, wherein during the catalytic dehydration step water is removed from the reaction mixture. Herein, it is preferred that water is removed by membrane separation. The invention further concerns the use of a membrane reactor for performing a process for converting ethanol to ethylene, wherein in the membrane reactor ethanol is dehydrated to form ethylene and water, and water is removed by membrane separation.

## Description

### Introduction

The present invention is in the field of chemical processing, in particular in the production of ethylene from ethanol.

### Introduction

Ethylene is a building block for the chemical industry. Most of the ethylene used comes from non-renewable resources, which have known negative environmental impact. A source with less negative environmental impact is sought for. One of the options is ethanol which can be obtained from biomass. Currently ethanol is used in the transport sector, as a biofuel additive for gasoline. Due to the electrification of the transport sector, a surplus of ethanol is expected to occur. There is therefore need for a new destination of the current ethanol production capacity.

Processes for converting ethanol into ethylene are described by Mohsenzadeh et al. (ChemBioEng Rev, 4, No. 2, p1-18, 2017) and Yakovleva et al. (Catalysis in Industry, Vol. 8 No. 2, p152-167, 2016). In these papers different catalyst and reactor designs for the catalytic dehydration of ethanol to ethylene are described. Catalytic dehydration of ethanol to ethylene concerns the following overall reaction equation:
catalyst

C₂H₅OH → C₂H₄ + H₂O (1)

Typically ethanol is converted in a cascade of adiabatic fixed bed reactors into ethylene. To perform the catalytic dehydration on commercial scale, several thermodynamic considerations need to be taken into account. The catalysed reaction is an endothermic reaction and consequently a higher process temperature provides for a higher yield. Moreover, during the reaction more species are formed than disappear (ethylene and water from ethanol) and therefore a low pressure is preferred. Typically, the reaction is therefore performed at a pressure in the range from 1 - 5 bar and 300 - 450°C. The reaction product of the catalytic dehydration needs to undergo a product recovery step and a ethylene purification step to obtain ethylene of sufficient purity.

Low temperatures are preferred for the product recovery step as the major contaminants (water and ethanol) have a lower boiling temperature than ethylene. By reducing the temperature to 40 - 50°C these contaminants liquefy which makes separation of the gaseous ethylene straightforward. Also higher pressures are preferred as the remnant minor contaminants can only be removed in an ethylene purification step by cryogenic distillation. For distillation the ethylene needs to be liquid, which requires a low temperature and a high pressure. Typically for cryogenic distillation the temperature is brought back to about minus 20°C to minus 30°C and the pressure is raised to 20 to 30 bar.

Consequently the process design comprises cooling and compression steps after reaction. Already the cooling steps are not optimal efficient as not all of the heat obtained can be re-used. However the major cost for product purification, both from OPEX and CAPEX perspective, are the gas compressors that are needed to increase the pressure of the gaseous ethylene. In addition to the cost perspective these steps also have a negative environmental impact of the process, as the energy needed cannot always be retrieved from renewable sources.

In addition, although high temperature during reaction increases the yield, a high temperature of the reaction step also has drawbacks. Obviously energy is needed to preheat the reactors and the incoming flows, but also coke-deposits is a problem. Due to the coke-deposition regular regeneration of the catalyst is needed which reduces the efficiency of the process. Part of the heating of the incoming flow typically is in furnaces which are not suitable for re-using recovered heat from the downstream cooling steps.

In the art the optimization of the catalytic dehydration process has been aimed at improving the raw material (the ethanol), the placement of the catalyst in the reaction (fixed-bed or fluidized bed), catalyst selection and integration with ethanol production.

The vast majority of the processes concern alumina catalysts. In contrary WO 2007063281 A1 concerns a heteropolyacid catalyst. Such catalyst have relative low selectivity and cannot be used above 250°C because of their low thermal stability in reductive media. In addition, heteropolyacid catalyst are deactivated in the presence of water, which makes them less attractive for (bio)ethanol dehydration. Consequently in WO 2007063281 A1 ethanol comes from syngas and the side reaction products being ethyl ethers needs to be recycled. Water is removed after the reaction.

US4396789A concerns converting ethanol into ethylene at temperatures above 400°C and a pressure from 20 to 40 bar. Water is removed after the reaction. US 2017/0266635 A1 concerns converting ethanol into ethylene wherein the ethanol is diluted with an highly heated inert gas before reaction. The reaction temperature is above 300°C and the demonstrated pressure is around 5 bar. Water is removed after the reaction.

There is need for a catalytic dehydration process for converting ethanol in ethylene that is more cost efficient and environmental friendly. There is a need for a process that has a lower CAPEX, OPEX and/or energy usage. There is need for a process that reduced the frequency for catalyst regeneration and/or catalyst lifetime. There is need of a process with improved reactor design providing for lower spatial requirements or higher conversion per volume.

### Summary of the invention

The inventors surprisingly found that a process for converting ethanol to ethylene can be improved by removing water from the reaction mixture during the catalytic dehydration step. The improvement results in higher cost efficiency and lowers environmental impact. By removing water during the catalytic dehydration step the need for a low pressure and a high temperature during this step is no longer there as the reaction balance shifts to the reaction products. Consequently, before entering the catalytic dehydration reactor, liquid ethanol can be pressurized to a higher pressure compared to a conventional process. This reduces or even prevents costly pressurization of gaseous ethylene after the reaction. Next to this, the catalytic dehydration reaction can be operated at a lower temperature, which provides for lower energy usage and is beneficial for preventing the significant problem of coke formation and/or increasing catalyst lifetime. Additionally, by removing water, the catalytic dehydration reaction (1) is forced to the right side of the equation, therewith increasing the reaction rate which makes process intensification possible. Finally since less heating an cooling is required a more efficient heat exchange between the heating sections and the cooling sections of the process can be realized.

By removing water, the reaction conditions and catalyst can be selected that deliver high yield and selectivity. The process of the invention is therefore more versatile.

### Detailed Description

The invention relates in a first aspect to a process for converting ethanol to ethylene, comprising a catalytic dehydration step wherein ethanol is dehydrated to form ethylene, wherein during the catalytic dehydration step, water is removed from the reaction mixture. The invention furthermore concerns the use of a membrane reactor for performing a process for converting ethanol to ethylene, wherein in the membrane reactor ethanol is dehydrated to form ethylene and water, and water is removed by membrane separation. All embodiments as described in this document directed to the process for converting ethanol to ethylene also apply for the use of a membrane reactor for performing a process for converting ethanol to ethylene and vice versa.

### Reaction and reactants

The invention concerns a catalytic dehydration wherein ethanol is dehydrated to form ethylene. Ethylene is also known as ethene. During catalytic dehydration, water is split of the ethanol and ethylene is formed. Preferably the yield of the catalytic dehydration of ethanol to ethylene of the invention is above 90 mole%, more preferably above 95 mole%, most preferably above 98 mole%. The yield is the molar ratio of ethylene formed over the ethanol fed, expressed in percentage. Two parallel routes have been identified for forming ethylene, either ethylene is directly formed or first diethyl ether as an intermediate product is formed. Diethyl ether is also known as ethoxyethane. Possible side products of the reaction are diethyl ether, acetaldehyde and butylene. Preferably the selectivity of the catalytic dehydration of ethanol to ethylene of the invention is above 70 mole%, more preferably above 80 mole%, even more preferably above 90 mole%, even more preferably above 95 mole%, most preferably above 98 mole%. The selectivity is the molar ratio of the ethylene formed over the sum of the products formed, expressed in percentage.

In a preferred embodiment of the invention, the ethanol is obtained from fermentation of renewable materials or from thermochemical conversion of renewable materials. Preferred sources of renewable materials are crops such as wheat, maize, sugar beet and sugar cane, lignocellulosic materials such as wood and herbaceous biomass, and waste streams such as food waste, municipal solid waste, agricultural waste and paper waste.

During fermentation, sugars are converted into ethanol by microorganisms. More complex biomaterials, such as starches and lignocellulosic material, need a pre-treatment, such as hydrolysis before fermentation. For thermochemical conversion of renewable materials, these materials are formed into to syngas and then the syngas is either fermented to ethanol or catalytically converted to ethanol.

In a preferred embodiment of the invention, the feedstock for the catalytic dehydration step comprises at least 50 wt.% ethanol based on total weight of the feedstock, more preferably at least 75 wt.%, even more preferably at least 90 wt.%, even more preferably at least 95 wt.% most preferably at least 98 wt.%. A higher share of ethanol improves efficiency of the process for converting ethanol to ethylene.

Often the ethanol obtained from fermentation of renewable materials or from thermochemical conversion of renewable materials comprises water. Considering water and ethanol form an azeotrope, separation ethanol from water is energy intensive and difficult. As separation of water and ethylene according to the invention is more straightforward, some water may remain in the feedstock. This may benefit the overall process efficiency from renewable material to ethylene. Preferably the feedstock for the catalytic dehydration step comprises 2.0 - 50 wt.% water on total weight of the feedstock, more preferably 1.0 - 25 wt.%, even more preferably 0.5 - 10 wt.%, most preferably 0.1 -2.0 wt.%.

### Catalyst

Catalysts for catalytic dehydration of ethanol to ethylene are described by Mohsenzadeh et al. (ChemBioEng Rev, 4, No. 2, p1-18, 2017) and Yakovleva et al. (Catalysis in Industry, Vol. 8 No. 2, p152-167, 2016). The performance of these catalysts with regards to yield and selectivity as a function of feedstock composition, temperature and pressure are known in the art.

In a preferred embodiment the catalyst for catalytic dehydration of ethanol to ethylene is selected from the list consisting of acid-based catalysts, oxide catalysts and molecular sieves. More preferably the catalyst is an oxide catalyst or a molecular sieve, most preferably the catalyst is an oxide catalyst. Preferably the oxide catalyst is an alumina oxide catalyst. Preferably the molecular sieve catalyst is a ZSM-5 catalyst or a modification thereof or a SAPO-34 catalyst or a modification thereof. Acid-based catalysts are phosphoric acid catalysts or heteropolyacid catalysts. Preferably the acid-based catalyst is a phosphoric acid catalyst. Considering heteropolyacid catalysts provide for a low selectivity, preferably the catalysts is not a heteropolyacid catalyst.

Water removal from the reaction mixture during the catalytic dehydration step wherein ethanol is dehydrated to form ethylene is beneficial regardless of the type of catalyst used. The benefits as sketched apply for any catalyst since water removal has a positive effect on the reaction equilibrium whilst the catalyst concerns the rection kinetics.

### Pressurization

In a preferred embodiment the feedstock for the catalytic dehydration step is in liquid form when pressurized, preferably the liquid feedstock is brought to a pressure in the range of 5 - 40 bar, more preferably 10 - 40 bar, even more preferably 15 - 40 bar, even more preferably 20 - 40 bar, even more preferably 20 - 35 bar, most preferably 20 - 30 bar. Preferably after the pressurization the liquid feedstock is heated to a temperature in the range of 100 - 400 °C, more preferably 125 - 400 °C, even more preferably 150 - 300 °C, most preferably 150 - 250 °C. Preferably the feedstock is heated in a heat exchanger, preferably the feedstock is heated in a heat exchanger with steam as the heating medium.

### Reaction conditions

The following preferred embodiments apply for the catalytic dehydration step.

In a preferred embodiment the pressure is in the range of 5 - 40 bar, more preferably 10 - 40 bar, even more preferably 15 - 40 bar, even more preferably 20 - 40 bar, even more preferably 20 - 35 bar, most preferably 20 - 30 bar. Considering the preferred pressure of the purification step is above 15 bar, a higher pressure of the output of the catalytic dehydration step reduces or even takes away the need for costly compression of the gaseous output stream. Instead the liquid feedstock of the catalytic dehydration step is pressurized before the feedstock is vaporized. Pressurizing of a liquid is less costly compared to pressurizing a gas.

In a preferred embodiment the reaction temperature is in the range of 100 - 400 °C, more preferably 125 - 400 °C, even more preferably 150 - 300 °C, most preferably 150 - 250 °C. This reaction temperature range is a balance between the reaction rate which increase with temperature, and undesirable coking which also increase with temperature. Considering this reaction temperature is lower than in a conventional process, less heating is required. This is especially beneficial considering typically the reaction mixture is brought to the reaction temperature in a furnace, which are energy inefficient.

In a preferred embodiment for the catalytic dehydration step the pressure is in the range of 10 - 40 bar and the reaction temperature is in the range of 150 - 400 °C, preferably the pressure is in the range of 15 - 40 bar and the reaction temperature is in the range of 150 - 300 °C, more preferably the pressure is in the range of 20 - 40 bar and the reaction temperature is in the range of 150 - 250 °C.

### Water removal

In a preferred embodiment during the catalytic dehydration step water is removed from the reaction mixture by membrane separation. Water permeates the membrane whilst the ethylene and ethanol remain in the reaction mixture. The membrane for removal of water (herein also referred to as the membrane) may have different spatial configurations. Preferably the membrane is selected from a tubular membrane, a sheet and a monolith, more preferably the membrane is a tubular membrane. All of these spatial membrane configurations are known to withstand the elevated pressure of the current invention.

The temperature and pressure during the catalytic dehydration step is such that not all membrane materials are suitable. Some membranes materials decompose at elevated temperatures or are too fragile for elevated pressures. The membrane for removal of water can accordingly suitably function at these reaction conditions. Therefore, the membrane for removal of water is preferably a hydrophilic membrane. Preferably the membrane is a zeolite membrane, a metal-organic framework (MOF) membrane, an amorphous membrane or a polymer membrane. Van Kampen et al. (Chemical Engineering Journal 374 (2019) 1286-1303) provides an overview of several zeolite, amorphous and polymer membranes that are selective towards water permeation at temperatures between 200 and 400°C.

Preferred zeolite membranes are mordenite (MOR), ZSM-5, chabazite (CHA), silicalite-1 (SIL-1), Z4A, faujasite (FAU), Si/AI variant MFI membranes, and the like.

Preferred amorphous membranes are (amorphous) silica membranes and organic-inorganic hybrid silica membranes. Preferred organic-inorganic hybrid silica membranes are HybSi membranes (known from e.g. Castricum et al. J. Mater. Chem. 2008, 18, 2150-2158 and Agirre Arisketa et al. Sep. Purif. Technol. 2014, 121, 2-12), APTES-PA (Aminopropyl triethoxysilane-Polyamide) membranes and BTESE (1, 2-bis (triethoxysilyl) ethane) membranes.

The polymer membranes preferably comprise ceramic-supported polymers (CSP), as these are particularly suitable for functioning at elevated temperatures. Preferred polymer membranes are polyvinyl alcohol (PVA) based polymeric membranes, polyimide (PI) based membranes, Polybenzimidazol (PBI) based membranes, Polydimethylsiloxane (PDMS) based membranes, and sulfonated polymeric membranes (e.g. SPEEK or Nafion-based such as Li-Nafion).

Membranes that are used for the water removal in Fischer-Tropsch processes may also be suitably used for the present invention (see e.g. Rohde et al. Microporous and Mesoporous Materials 115 (2008) 123-136). The skilled person may further find guidance in e.g. Smitha et al., J. Membr. Sci. 2004, 241, 1, 1-21 and US 2021/0046442 A1 for suitable membrane selection.

Several membranes were tested for water separation during methanol synthesis from CO₂ and H₂ at 35 bar and temperatures between 150°C and 325°C (M. Saric, R. Sumbharaju, M. van Tuel , P. Marcelis, Membrane reactor to enhance a methanol production from CO2 and H2 in biomass to biodiesel route, presented at Euromembrane 2021 in Copenhagen). Polymer membranes such as SPEEK, PI, PDMS, Nafion membranes and amorphous membranes such as APTES-PA and BTESE membranes were all found to be suitable. Considering this methanol synthesis is operated at a same temperature and pressure range as the current conversion from ethanol to ethylene and the reaction species are very similar these membranes are also suitable for the current ethanol to ethylene process. Considering ethanol and ethylene are larger molecules than methanol, water separation for the current ethanol to ethylene process is more straightforward compared to methanol synthesis. Preferably for the current invention the membrane is selected from a polymer membrane and an amorphous membrane, more preferably the membrane is selected from an APTES-PA, SPEEK, PI, PDMS, Nafion and BTESE membrane.

In a preferred embodiment the membrane does not catalyze the catalytic dehydration of ethanol to ethylene or in other words the membrane is inert. It is believed that having the reaction taking place on the membrane surface and/or in the membrane pores affect the separation selectivity in a negative manner. More ethanol or ethylene may permeate the membrane and/or less water permeates the membrane. Moreover the reaction rate may be lower compared to a configuration in which the membrane and the catalyst are separate. Typically the latter configuration in which the membrane and the catalyst are separate provides for more catalytic surface and less diffusion limitation. In other words, in this preferred embodiment the membrane is not the catalyst for the catalytic dehydration of ethanol to ethylene.

For the preferred embodiment wherein water is removed from the reaction mixture by membrane separation the catalytic dehydration step is carried out in a membrane reactor. Membrane reactors are known in the art. Preferably the membrane reactor is a fixed bed membrane reactor or a fluidized bed membrane reactor. The bed of the fixed bed or the fluidized bed refers to the catalyst bed. For the current invention the reaction mixture enters the reactor on the retentate side and water that is formed permeates the membrane. The catalyst is present on the retentate side. The reaction mixture exits the membrane reactor on the retentate site.

In a preferred embodiment the water content of the reaction product of the catalytic dehydration step is below 20 wt.% based on total weight of this reaction product, preferably below 10 wt.%, more preferably below 5 wt.%, most preferably below 1 wt.%. In case no water would be removed and the feed comprises 100 wt.% ethanol and the conversion is 100 %, the water content of the reaction product would be about 39 wt.% on total weight of the reaction product. The reaction product of the catalytic dehydration step is gaseous.

### Ethylene recovery step

In a preferred embodiment the process for converting ethanol to ethylene comprises an ethylene recovery step. In the ethylene recovery step major contaminants such as ethanol, CO₂ and water are removed from the reaction product of the catalytic dehydration step. The product of the ethylene recovery step typically comprises more than 95.0 wt.% ethylene on total weight of the product, preferably more than 98.0 wt.% even more preferably more than 99.0 wt.%.

Preferably the ethylene recovery step comprises a CO₂ removal step, one or more cooling steps and/or one or more water removal steps. One or more cooling step may be combined with one or more water removal step. During water removal also remnant ethanol and some minor components may be removed.

In a preferred embodiment the ethylene recovery step comprises one or more water removal steps wherein water is removed by cooling the reaction product of the catalytic dehydration step to a temperature at which water liquifies and the liquid water is removed from the reaction product. More preferably the ethylene recovery step comprises two or more removal step wherein water is removed by cooling the reaction product of the catalytic dehydration step to a temperature at which water liquifies and the liquid water is removed from the reaction product, wherein the reaction product temperature is lowered between the two or more water removal steps. Preferably for this embodiment the reaction product is cooled to 50°C or lower, more preferably 40°C or lower, even more preferably 30°C or lower, most preferably 20°C or lower. Preferably for this embodiment the reaction product is cooled in a heat exchanger or a direct cooler.

In a preferred embodiment the reaction product of the catalytic dehydration step is cooled in a heat exchanger or a direct cooler. In a heat exchanger the reaction product is not in direct contact with the cooling medium, in a direct cooler the reaction product is in direct contact with the cooling medium. Preferably the cooling medium of the direct cooler is water or air. Preferably the cooling medium of the heat exchanger is water or steam. Preferably the reaction product is cooled to 50°C or lower, more preferably 40°C or lower, even more preferably 30°C or lower, most preferably 20°C or lower. The reaction product preferably is cooled in 1 to 3 of these cooling steps, more preferably 1 or 2 of these cooling steps, most preferably in 1 cooling step. In case water liquefies upon cooling, the liquid water is removed from the reaction product. In case the cooler is a direct cooler preferably the direct cooler comprises a drain possibility, preferably at the bottom of the direct cooler; in case the cooler is a heat exchanger preferably a knock-out drum is placed after the heat exchanger. More preferably in case the cooler is a direct cooler the direct cooler comprises a drain possibility preferably at the bottom of the direct cooler, in case the cooler is a heat exchanger preferably a knock-out drum is placed after the heat exchanger. In one preferred embodiment the direct cooler comprises a demister on top of the direct cooler.

In a preferred embodiment the ethylene recovery step does not comprise quenching. During quenching water vapour comprised in the reaction product condenses on liquid water droplets provided to the reaction product. A conventional process for converting ethanol to ethylene typically comprises a quenching step. However considering the water content of the reaction product of the invention is lower than that of a conventional process a quenching step may not be needed. Quenching has drawbacks as through quenching additional water is introduced and any heat recovered from the reaction product cannot be reused.

Compared to a conventional process for converting ethanol to ethylene the reaction product of the catalytic dehydration step of the invention comprises less water. Therefore the OPEX and CAPEX for water removal advantageously will be lower for the process according to the invention.

In a preferred embodiment the ethylene recovery step comprises a CO₂ removal step. Preferably the CO₂ removal step is performed in a caustic wash column. In a preferred embodiment the ethylene recovery step comprises an additional water removal step wherein water is removed by contacting the reaction product with molecular sieves. Typically contacting the reaction product with molecular sieves is done on a reaction product that has a low water content. As some water is introduced by caustic washing, preferably the ethylene recovery step comprises a CO₂ removal step which is performed in a caustic wash column followed by a water removal step wherein water is removed by contacting the reaction product with molecular sieves.

### Ethylene purification step

In a preferred embodiment the process for converting ethanol to ethylene comprises an ethylene purification step. This step is after the ethylene recovery step. During the ethylene purification step minor contaminants are removed from the reaction product. The product of the ethylene purification step typically comprises more than 99.5 wt.% ethylene on total weight of the product, preferably more than 99.7 wt.% even more preferably more than 99.9 wt.%.

Preferably the ethylene purification step comprises a cryogenic distillation step. Preferably the cryogenic distillation is performed at a pressure of 15 - 40 bar, more preferably at a pressure of 20 - 35 bar, even more preferably at a pressure of 20 - 30 bar. Preferably the cryogenic distillation is performed at a temperature of -10°C to -40 °C, more preferably at a temperature of -20°C to -30°C.

### Process integration

An advantage of the inventive process for converting ethanol to ethylene is that the catalytic dehydration step can be performed at a higher pressure. This reduces or eliminates the need for expensive and energy-inefficient compression of the gaseous reaction product of the catalytic dehydration step as the reaction product is already at the preferred pressure of the ethylene purification step. Preferably during the ethylene recovery step the pressure of the reaction product of the catalytic dehydration step increases with 5 to 15 bar, preferably with 3 to 10 bar, more preferably with 1 to 5 bar, most preferably there is no pressure increase.

In a preferred embodiment the process for converting ethanol to ethylene does not comprise a compression step of the reaction product of the catalytic dehydration step. However there may be situations that for overall process efficiency reasons still some compression is required before the ethylene purification step. For example upon cooling of the reaction product of the catalytic dehydration step the pressure of the reaction product drops to some extend. In a preferred embodiment the ethylene recovery step comprises one or more compression steps. Preferably at the end of the one or more compression steps the pressure of the reaction product of the catalytic dehydration step is increased with 5 to 15 bar, preferably with 3 to 10 bar, more preferably with 1 to 5 bar. Preferably the one or more compression steps bring the pressure of the reaction product of the catalytic dehydration step to 15 - 40 bar, even more preferably to 20 - 35 bar, most preferably to 20 - 30 bar.

Due to the water removal the equilibrium of the catalytic dehydration step shifts to the reaction product, regardless of the catalyst and reaction conditions. Consequently there is more freedom in selecting the catalyst and the reaction conditions of the catalytic dehydration step towards optimal selectivity, conversion and overall process efficiency. For example inefficient recycle of the part of the reaction product of the catalytic dehydration step may be prevented. The recycle may be needed for reactions with low selectivity. In a preferred embodiment the process for converting ethanol to ethylene does not comprise a recycle flow wherein part of the reaction product of the catalytic dehydration step is recycled to the feed of the catalytic dehydration step.

### General Definitions

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value more or less 1% of the value.

The present invention has been described above with reference to a number of exemplary embodiments. Modifications and alternative implementations of some parts or elements are possible, and are included in the scope of protection as defined in the appended claims. All citations of literature and patent documents are hereby incorporated by reference.

Bara stands for bar absolute and concerns the pressure that is zero-referenced against a perfect vacuum. 1 bar is 100,000 N/m². 1 bar is 100,000 Pascal.

### Description of the figure

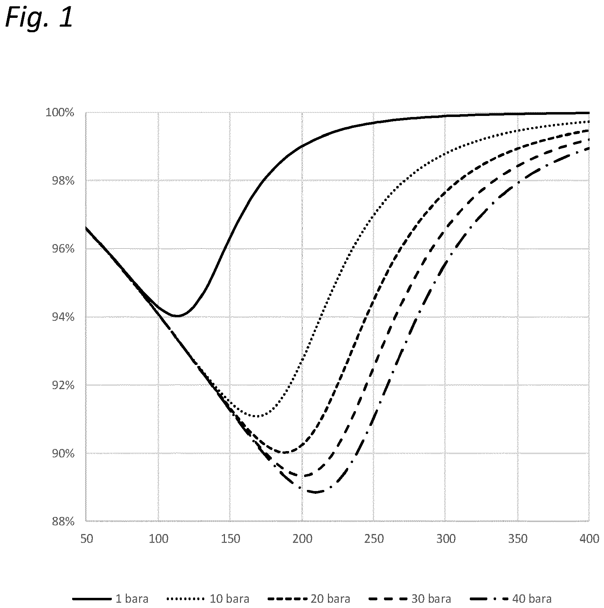

Figure 1 depicts the ethanol to ethylene conversion as a function of temperature and pressure, as predicted by ASPEN simulations. The figure concerns an equilibrium situation so without water removal. The X-axis is the temperature expressed in °C, the Y-axis is the ethanol conversion expressed in %.

### Examples

### Example 1 - Conversion as a function of pressure and temperature

A model simulation was performed (ASPEN Plus 36.0 - equilibrium based model) in which the conversion of the dehydration of ethanol to ethane at different temperatures and pressures was simulated.

C₂H₅OH (g) ↔ C₂H₄ (g) + H₂O (g)

For the model simulation water removal was not included. The Peng-Robinson Equation of State was used for the gas phase, NRTL activity coefficient model for the liquid phase. The simulation was equilibrium-based with the use of RGibbs reactos (Rigorous reaction and/or mutiphase equilibrium based on Gibbs free energy minimization).

The model demonstrates the Le Chatelier principle that for an endothermic reaction that has more species on the right side of the reaction equation the conversion increases with temperature and decrease with pressure. At 1 bar (solid line) already at 170°C a conversion of 98% can be obtained whilst at 40 bar (line with dots and stripes) the temperature needs to be at least 350°C. As generally a higher conversion is needed, also at 1 bar the reaction temperature is typically selected to be above 300°C. By removing one of the species on the right side also at higher pressures and lower temperatures a high conversion can be reached.

### Example 2 - CAPEX and OPEX of reaction product compression

A OPEX and CAPEX comparison was made for the cooling and compression costs for cooling and compression of the reaction product in a conventional process as published by Mohsenzadeh et al. (ChemBioEng Rev, 4, No. 2, p1-18, 2017) and the process of the invention. The compression train was simulated using Aspen Plus V10 and the equipment costs were calculated using Aspen Process Economic Analyzer Package. The assumptions for the OPEX calculations were 8000 operating hours per year, electricity cost: 0.1 €/kWh and cooling water cost: 0.01 €/ton, while for the CAPEX calculations are installation factor: 4 and depreciation time: 10 years. Process parameters were taken from by Mohsenzadeh et al. The Mohsenzadeh process comprises 3 cooling and compression steps bringing the gaseous reaction product from about 1 bar to 27 bar and from about 90°C to 15°C.

OPEX (table 1) and CAPEX costs (table 2) for compression and cooling of the reaction product was assessed as a function of the pressure of the reaction product leaving the reactor, targeting 27 bar pressure after the compression step. Clearly cost of compression heavily outweigh the cost for cooling. The largest saving can be obtained in case no further compression is needed; about 5000 kEuro in CAPEX (no installation costs included for this amount) and 2500 kEuro in OPEX. Scenario's in which less compression is needed are also in the tables; for example increasing of the pressure of the reaction product from 1 bar (base case) to 15 bar provides for about 4000 kEuro in CAPEX and 2100 kEuro in OPEX savings.

A large part of the operational cost concern energy costs for compression and cooling. Having a pressurized reaction product therefore also saves a considerable amount of energy.

**Table 1. OPEX costs in kEuros/year for compression and cooling of the reaction product.**

| | **Conventional** | **Process Intensification** | | |
|---|---|---|---|---|
| *Pressure of the reaction product* | *1 bar* | *5 bar* | *10 bar* | *15 bar* |
| **Total OPEX compression** | 2.554 | 1.318 | 734 | 420 |
| **Total OPEX cooling** | 27 | 13 | 8 | 5 |
| **Total OPEX** | 2.582 | 1.331 | 741 | 425 |
| **Savings** | | 1.250 | 1.840 | 2.157 |

**Table 2. CAPEX costs in kEuros (unless stated otherwise) for compression and cooling of the reaction product.**

| | **Conventional** | **Process Intensification** | | |
|---|---|---|---|---|
| *Reaction product pressure* | *1 bar* | *5 bar* | *10 bar* | *15 bar* |
| **Compressors** | 4.968 | 1.473 | 1.285 | 1.025 |
| **Coolers** | 64 | 31 | 17 | 12 |
| **Drums** | 28 | 5 | 5 | 5 |
| **Total costs** | 5.060 | 1.509 | 1.307 | 1.042 |
| **Total costs with installation** | 20.238 | 6.034 | 5.229 | 4.170 |
| **Savings** | | 14.204 | 15.009 | 16.068 |
| **Savings (k€/year) ^{∗}** | | 1.420 | 1.501 | 1.607 |

| | | | | |
|---|---|---|---|---|
| ^{∗} assuming 10 year depreciation time | | | | |

## Claims

1. A process for converting ethanol to ethylene, comprising a catalytic dehydration step wherein ethanol is dehydrated to form ethylene, wherein during the catalytic dehydration step water is removed from the reaction mixture.

2. The process according to claim 1, wherein water is removed by membrane separation.

3. The process according to claim 2, wherein the membrane separation is performed using a membrane selected from the list consisting of silica membranes, zeolite membranes, metal-organic framework (MOF) membranes and perovskite membranes, preferably the membrane is a zeolite membrane.

4. The process according to claim 2 or 3, wherein the membrane separation is performed using a membrane which does not catalyze the catalytic dehydration of ethanol to ethylene.

5. The process according to any one of claims 2 - 4, wherein the membrane separation is performed using a membrane selected from a tubular membrane, a sheet, a monolith, preferably wherein the membrane is a tubular membrane.

6. The process according to any one of the preceding claims, wherein for the catalytic dehydration step the reaction temperature is in the range of 150 - 400 °C, preferably in the range of 150 - 300 °C, more preferably in the range of 150 - 250 °C.

7. The process according to any one of the preceding claims, wherein for the catalytic dehydration step the pressure is in the range of 10 - 40 bar, preferably in the range of 15 - 40 bar, more preferably in the range of 20 - 40 bar.

8. The process according to any one of the preceding claims, wherein the water content of the reaction product of the catalytic dehydration step is below 20 wt.% based on total weight of this reaction product, preferably below 10 wt.%, more preferably below 5 wt.%, most preferably below 1 wt.%.

9. The process according to any one of the preceding claims, further comprising an ethylene recovery step, preferably wherein the ethylene recovery step comprises a CO₂ removal step and/or one or more water removal steps.

10. The process according to claim 9, wherein during the ethylene recovery step the pressure of the reaction product of the catalytic dehydration step increases with 5 to 15 bar, preferably with 3 to 10 bar, more preferably with 1 to 5 bar, or wherein there is no pressure increase of the reaction product of the catalytic dehydration step during the ethylene recovery step.

11. The process according to any one of the preceding claims, further comprising an ethylene purification step, preferably wherein the ethylene purification step comprises a cryogenic distillation step.

12. The process according to claim 11, wherein the ethylene purification step comprises a cryogenic distillation step and wherein the cryogenic distillation is performed at a pressure of 15 - 40 bar, more preferably at a pressure of 20 - 35 bar, even more preferably at a pressure of 20 - 30 bar.

13. The process according to any one of the preceding claims, wherein the catalytic dehydration step is performed with a catalyst selected from the list consisting of acid-based catalysts, oxide catalysts and molecular sieves.

14. Use of a membrane reactor for performing a process for converting ethanol to ethylene, wherein in the membrane reactor ethanol is dehydrated to form ethylene and water, and water is removed by membrane separation.

15. The use according to claim 14, wherein the membrane reactor is operated at a pressure in the range of 10 - 40 bar and a temperature in the range of 150 - 400 °C, preferably at a pressure in the range of 15 - 40 bar and a temperature in the range of 150 - 300 °C, more preferably at a pressure in the range of 20 - 40 bar and a temperature in the range of 150 - 250 °C.
